(19) Europäisches Patentamt
European Patent Office
Office européen des brevets

(11) Veröffentlichungsnummer : **0 021 074**
**B1**

(12) # EUROPÄISCHE PATENTSCHRIFT

(45) Veröffentlichungstag der Patentschrift : **28.04.82**

(51) Int. Cl.³ : **C 07 C 47/21**, C 07 C 45/51

(21) Anmeldenummer : **80102940.6**

(22) Anmeldetag : **27.05.80**

(54) **Verfahren zur Herstellung von Citral.**

(30) Priorität : **30.06.79 DE 2926562**

(43) Veröffentlichungstag der Anmeldung : **07.01.81 (Patentblatt 81/01)**

(45) Bekanntmachung des Hinweises auf die Patenterteilung : **28.04.82 Patentblatt 82/17**

(84) Benannte Vertragsstaaten : **BE CH DE FR GB IT LI NL**

(56) Entgegenhaltungen :
**AT - B - 342 016**
**DE - A - 2 411 530**
**DE - B - 2 157 035**
**DE - B - 2 657 335**
**FR - A - 2 274 590**
**US - A - 3 978 092**

(73) Patentinhaber : **BASF Aktiengesellschaft**
**Carl-Bosch-Strasse 38**
**D-6700 Ludwigshafen (DE)**

(72) Erfinder : **Nissen, Axel, Dr.**
**Panoramastrasse 51**
**D-6906 Leimen (DE)**
Erfinder : **Rebafka, Walter, Dr.**
**Schuetzenstrasse 4**
**D-6901 Eppelheim (DE)**
Erfinder : **Aquila, Werner, Dr.**
**Meissener Weg 35**
**D-6800 Mannheim 42 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 021 074 B1

Verfahren zur Herstellung von Citral

Die vorliegende Erfindung betrifft ein verbessertes Verfahren zur Herstellung von Citral (I ; 3,7-Dimethylocta-2,6-dien-1-al)

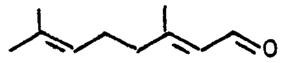

(I)

durch Erhitzen von Acetalen der allgemeinen Formeln IIa oder IIb

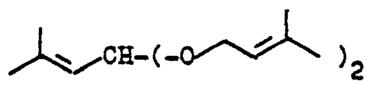

IIa

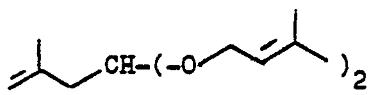

IIb

in Gegenwart von sauren Katalysatoren.

Diese Reaktion ist, sieht man von der erfindungsgemäßen Verbesserung ab, in ihren wesentlichen Merkmalen aus der DE-OS 24 11 530 bekannt. Hierbei handelt es sich um eine komplexe Umsetzung über drei Stufen, wie aus folgendem Reaktionsschema hervorgeht :

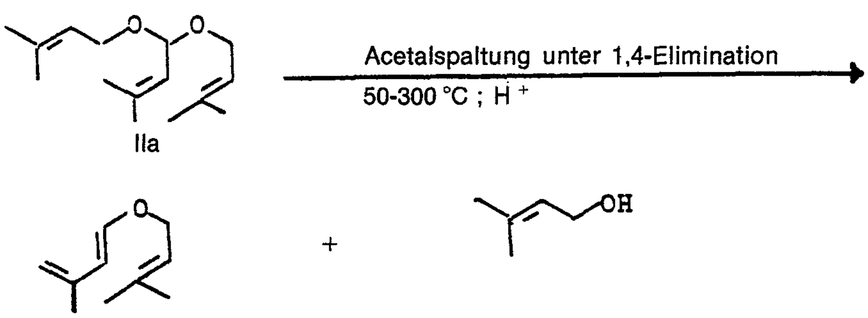


IIa

+

(V) (III)

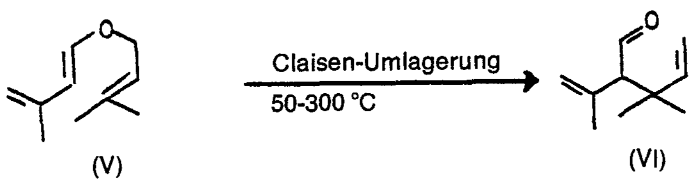


(V) (VI)

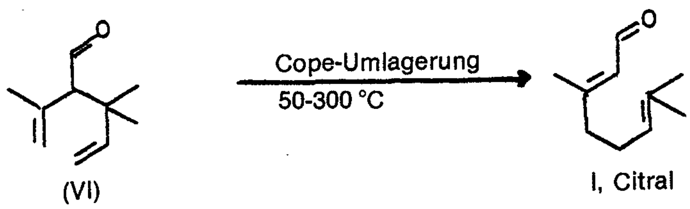


(VI) I, Citral

In ähnlicher Weise läuft die Reaktion ab, wenn man statt der Acetale IIa die isomeren Acetals IIb einsetzt.

Da sowohl die Ausgangsverbindungen IIa und IIb und das Verfahrensprodukt I als auch die Neben- und Intermediärprodukte III, V und VI empfindliche Substanzen sind, die in unerwünschter Weise reagieren können, ließen sich bisher an I nur Ausbeuten von bis zu 60-70 % erzielen.

Der Erfindung lag daher die Aufgabe zugrunde, die Ausbeute an I, dem wichtigen Duftstoff Citral, zu verbessern und somit das Verfahren insgesamt wirtschaftlicher zu gestalten.

Es wurde gefunden, daß man Citral (I)

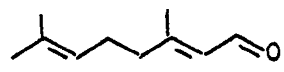

(I)

durch Erhitzen von Acetalen der allgemeinen Formeln IIa oder IIb

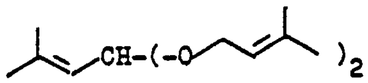

IIa

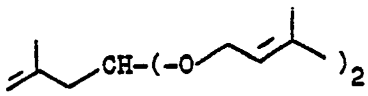

IIb

in Gegenwart von sauren Katalysatoren in hohen Ausbeuten erhält, wenn man das bei dieser Reaktion abgespaltene 3-Methylbut-2-en-1-ol (III, Prenol) laufend aus dem Reaktionsgemisch entfernt.

Es wurde weiterhin gefunden, daß man besonders gute Ergebnisse erzielt, wenn man die Reaktion in Gegenwart von 0,1-10 mol %, bezogen auf eingesetztes IIa bwz. IIb, einer inerten Flüssigkeit IV vornimmt, die unter dem Reaktionsdruck höher siedet als Prenol, jedoch tiefer als Citral und 2,8-Dimethyl-5-oxa-nona-1,3,7-trien (V) und 2,4,4-Trimethyl-3-formylhexa-1,5-dien (VI). Diese Flüssigkeiten IV werden im folgenden als « Zwischensieder » bezeichnet.

Die erfindungsgemäße laufende Entfernung des Prenol bewirkt eine Steigerung der Citral-Ausbeute auf etwa 85-90 %. Dieser Effekt ist als überraschend zu bezeichnen, da es sich bei der Herstellung von I nicht um eine Gleichgewichtsreaktion handelt, die durch Entfernung des Prenols aus dem Gleichgewicht begünstigt werden könnte. Besonders überraschend ist ferner, daß sich die Citral-Ausbeute bei Mitverwendung der Zwischensieder IV bis auf 95 % steigern läßt. Die Wirkungsweise der Zwischensieder, die definitionsgemäß höher sieden als Prenol (Sdp. 140 °C bei Normaldruck, wie im folgenden) aber tiefer als Citral (Sdp. 210 °C) und die Intermediärprodukte V (Sdp. 205 °C) und VI (Sdp. 200 °C), beruht vermutlich darauf, daß Citral, V und VI aufgrund der Partialdruckverhältnisse vorwiegend in der Flüssigphase verbleiben und daß der Partialdruck des Prenols herabgesetzt wird, wodurch dieses schnell und nahezu vollständig laufend entfernt werden kann. Das in die Gasphase gelangende Gemisch aus vorwiegend III und IV wird sodann in der Weise fraktioniert, daß III über Kopf der Fraktionierkolonne abgezogen wird, während der Zwischensieder IV in das Reaktionsgemisch zurückfließt oder in der Kolonne gehalten wird.

Man nimmt die Umsetzung vorzugsweise bei 100-160 °C und unter Normaldruck oder, wie es sich besonders empfiehlt, unter einem verminderten Druck von 40-140 mbar abs. vor, gemessen am Kopf der Fraktionierkolonne. Bei Normaldruck ist demnach ein Zwischensieder zu verwenden, der wie Diisoamyläther (Sdp. 172 °C), Anisol (Sdp. 155 °C) und Pseudocumol (Sdp. 169 °C) zwischen 140 und 190 °C siedet. Arbeitet man bei vermindertem Druck, so bleiben die Relationen der Siedepunkte der hier maßgebenden Substanzen in aller Regel gewahrt, so daß man beispielsweise auch in diesem Falle die vorgenannten Flüssigkeiten verwenden kann. Besonders gut geeignete Zwischensieder sind die Äther IVa und IVb

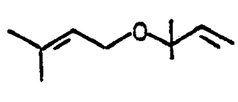

IVa, 3,3,7-Trimethyl-4-oxa-octa-1,6-dien

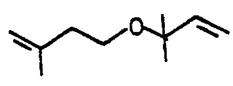

IVb, 3,3,7-Trimethyl-4-oxa-octa-1,7-dien

die man bei der Synthese von II aus Prenol und 3-Methyl-but-2-en-1-al als Nebenprodukte erhält.

Da die Zwischensieder die Aufgabe haben, die Entfernung des Prenols zu erleichtern, die Prenolkonzentration im Reaktionsgemisch indes wegen der erfindungsgemäßen Maßnahmen gering ist, braucht auch die Konzentration der Zwischensieder im flüssigen Reaktionsgemisch nicht sehr hoch zu sein. Vorzugsweise verwendet man die Zwischensieder IV daher in Mengen von 0,1-10, besonders 0,5-5 mol%, bezogen auf eingesetztes IIa bzw. IIb. Der überwiegende Teil des Zwischensieders befindet sich dabei während der Reaktion nicht im flüssigen Reaktionsgemisch sondern im unteren Bereich der Destillationskolonne.

Die dem Citral zugrundeliegenden Acetale IIa und IIb sind bekannt und in bekannter Weise durch Acetalisierung der Aldehyde VIIa und VIIb

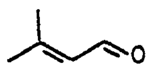 VIIa 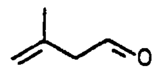 VIIb

mit Prenol (III) erhältlich.

Die Umsetzung von IIa bzw. IIb zu I wird in bekannter Weise in Gegenwart eines sauren Katalysators vorgenommen, wobei sich nichtflüchtige Protonensäuren wie Schwefelsäure, p-Toluolsulfonsäure und vor allem Phosphorsäure als besonders geeignet erwiesen haben. Die Menge dieser Säuren richtet sich nach ihrer. Stärke und beträgt im allgemeinen 0,001-0,5 Gew.%, bezogen auf die Gesamtmenge des Reaktionsgemisches. Im Falle der Verwendung von Phosphorsäure beträgt der bevorzugte Konzentrationsbereich 0,005-0,05 Gew.%. Häufig ist es zweckmäßig, den sauren Katalysator erst dann zuzusetzen, wenn eine Temperatur oberhalb des Siedepunktes des Prenols (III) erreicht ist. Dies empfiehlt sich vor allem, wenn man von prenolhaltigen Rohprodukten IIa und IIb ausgeht.

Es ist ein besonderer Vorteil des erfindungsgemäßen Verfahrens, daß sich die Mitverwendung von Lösungsmitteln erübrigt. Zwar kann man auch den Zwischensieder IV als Lösungsmittel betrachten, jedoch ist dessen erforderliche Menge wesentlich geringer als die Menge, in welcher Lösungs- und Verdünnungsmittel normalerweise verwendet werden, um unerwünschte Nebenreaktionen zu unterbinden. Will man neben dem Zwischensieder trotzdem ein Lösungsmittel mitverwenden, so empfehlen sich Lösungsmittel, welche höher als Citral sieden, z.B. hochsiedende Benzinfraktionen.

Die Menge des Lösungsmittels beträgt vorzugsweise das 0,5-3-fache des Volumens von IIa bzw. IIb.

Zur destillativen Entfernung des Prenols und zur gleichzeitigen Zurückhaltung der höher siedenden Komponenten des Reaktionsgemisches verwendet man eine Fraktionierkolonne mit zweckmäßigerweise 10-30 theoretischen Böden.

Da die Intermediärprodukte und das Citral sehr sauerstoffempfindlich sind und leicht polymerisieren, empfiehlt es sich, unter sorgfältigem Luftausschluß und unter Schutzgasatmosphäre, z.B. unter Stickstoff zu arbeiten. Zusätzlich ist es von Vorteil, die Reaktion in Gegenwart von $10^{-3}$-10 Gew.%, bezogen auf das eingesetzte Acetal IIa bzw. IIb, eines Polymerisationsinhibitors, vorzugsweise einer phenolischen Verbindung dieser Art, vorzunehmen. Derartige Inhibitoren sind beispielsweise Hydrochinon, Brenzkatechin, Trimethylhydrochinon, 2,6-Di-tert.-butyl-p-kresol und Tocopherol. Um auch Polymerisationen in der Fraktionierkolonne zu unterbinden, gibt man den Inhibitor zweckmäßigerweise in den Rücklauf am Kopf der Kolonne.

Beispiel 1

Ein Gemisch aus 2 000 g 98gew.%igem (Rest Prenol III = 3-Methylbut-2-en-1-ol) 3-Methylbut-2-en-al-diprenylacetal (IIa), 50 g 3,3,7-Trimethyl-4-oxa-octa-1,6-dien (als Zwischensieder IVa), 0,5 g 65gew.%iger Phosphorsäure und 2 g Hydrochinon wurde unter Stickstoffatmosphäre auf 145 °C erhitzt, wobei das abgespaltene Prenol laufend über eine Fraktionierkolonne mit 15 theoretischen Boden so abdestilliert wurde, daß der Zwischensieder in der Destillationskolonne gehalten wurde. Der Druck am Kopf der Kolonne betrug 90 mbar und die Temperatur 80-83 °C. Nach 6 Stunden war die Reaktion beendet, wie daran erkennbar war, daß die theoretische Menge an Prenol abgespalten und somit praktisch quantitativ zurückgewonnen war.

Die übliche destillative Aufarbeitung lieferte das Citral in einer Ausbeute von 96,6 %.

Unter den gleichen Bedingungen, jedoch mit Di-tert.-butyl-p-kresol als Inhibitor wurde eine Ausbeute von 95,3 % erzielt.

Ohne Mitverwendung des Zwischensieders betrug die Ausbeute an Citral auf 89,4 %, und ohne Zwischensieder und ohne Inhibitor 83,8 %.

Unter den im ersten Absatz dieses Beispiels genannten Bedingungen, also mit dem Zwischensieder und dem Inhibitor, jedoch unter Normaldruck und damit ohne laufende Entfernung des Prenols betrug die Ausbeute an Citral nur 52,2 %.

Beispiel 2

Unter den im ersten Absatz von Beispiel 1 genannten Bedingungen, jedoch ausgehend vom 3-Methyl-but-3-en-1-al-diprenylacetal (IIb) wurde das Citral in einer Ausbeute von 72 % erhalten.

**Ansprüche**

1. Verfahren zur Herstellung von Citral (I)

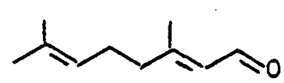 (I)

durch Erhitzen von Acetalen der allgemeinen Formeln IIa oder IIb

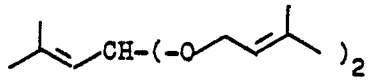

IIa

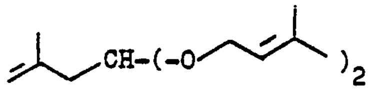

IIb

in Gegenwart von sauren Katalysatoren, dadurch gekennzeichnet, daß man das bei dieser Reaktion abgespaltene 3-Methylbut-2-en-1-ol (III, Prenol) laufend aus dem Reaktionsgemisch abdestilliert.

2. Verfahren nach Anspruch 1, dadurch gekennzeichnet, daß man die Reaktion in Gegenwart von 0,1-10 mol%, bezogen auf eingesetztes IIa bzw. IIb, einer inerten Flüssigkeit IV vornimmt, die unter dem Reaktionsdruck höher siedet als Prenol, jedoch tiefer als Citral und 2,8-Dimethyl-5-oxa-nona-1,3,7-trien (V) und 2,4,4-Trimethyl-3-formylhexa-1,5-dien (VI).

3. Verfahren nach Anspruch 2, dadurch gekennzeichnet, daß man als Flüssigkeit IV einen Äther der allgemeinen Formeln IVa oder IVb.

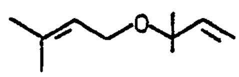

IVa

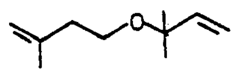

IVb

verwendet.

4. Verfahren nach den Ansprüchen 1 bis 3, dadurch gekennzeichnet, daß man unter Ausschluß von Sauerstoff arbeitet.

5. Verfahren nach den Ansprüchen 1 bis 4, dadurch gekennzeichnet, daß man die Umsetzung in Gegenwart eines phenolischen Polymerisationsinhibitors vornimmt.

**Claims**

1. A process for the preparation of citral (I)

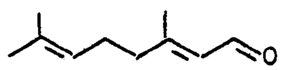

(I)

by heating an acetal of the general formula IIa or IIb

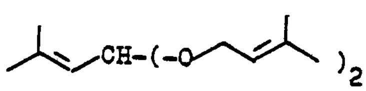

IIa

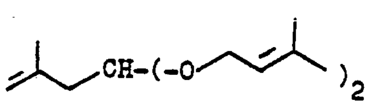

IIb

in the presence of an acid catalyst, characterized in that the 3-methylbut-2-en-1-ol (III, prenol) eliminated in the reaction is continuously distilled from the reaction mixture.

2. A process as claimed in claim 1, characterized in that the reaction is carried out in the presence of 0.1-10 mole%, based on IIa or IIb employed, of an inert liquid IV which under the reaction pressure boils at a higher temperature than prenol but at a lower temperature than citral, 2,8-dimethyl-5-oxa-nona-1,3,7-triene (V) and 2,4,4-trimethyl-3-formylhexa-1,5-diene (VI).

3. A process as claimed in claim 2, characterized in that an ether of the general formula IVa or IVb

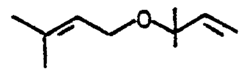

IVa

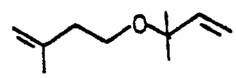

IVb

is used as the liquid IV.

4. A process as claimed in claims 1 to 3, characterized in that the reaction is carried out with exclusion of oxygen.

5. A process as claimed in claims 1 to 4, characterized in that the reaction is carried out in the presence of a phenolic polymerization inhibitor.

**Revendications**

1. Procédé de préparation de citral (I)

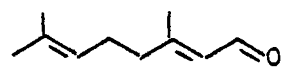

(I)

par chauffage d'acétals des formules générales IIa ou IIb

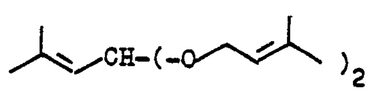

IIa

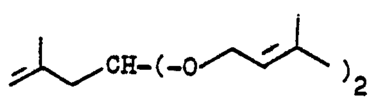

IIb

en présence de catalyseurs acides, caractérisé en ce que l'on élimine en continu le méthyl-3 butène-2 ol-1 (III, prénol) libéré pendant cette réaction du mélange réactionnel par distillation.

2. Procédé suivant la revendication 1, caractérisé en ce que la réaction est effectuée en présence de 0,1 à 10 % molaires, par rapport au composé IIa ou IIb mis en œuvre, d'un liquide IV inerte, dont le point d'ébullition sous la pression réactionnelle est supérieur à celui du prénol, mais inférieur à celui du diméthyl-2,8 oxa-5 nonatriène-1,3,7 (V) et du triméthyl-2,4,4 formyl-3 hexadiène-1,5 (VI).

3. Procédé suivant la revendication 2, caractérisé en ce que l'on emploie comme liquide IV un éther de l'une des formules générales IVa ou IVb.

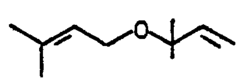

IVa

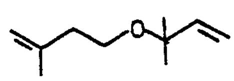

IVb

4. Procédé suivant l'une des revendications 1 à 3, caractérisé en ce que l'on travaille à l'abri de l'oxygène.

5. Procédé suivant l'une des revendications 1 à 4, caractérisé en ce que la réaction est réalisée, en présence d'un inhibiteur de polymérisation phénolique.